# EUROPÄISCHE PATENTSCHRIFT

(11) **EP 3 340 961 B1**
(45) Veröffentlichungstag und Bekanntmachung des Hinweises auf die Patenterteilung: **10.07.2019**
(21) Anmeldenummer: 16765929.1
(22) Anmeldetag: 23.08.2016
(51) Int. Cl.: A61J 15/00

(54) **MAGENSONDE**
GASTRIC PROBE
SONDE GASTRIQUE

(30) Priorität: 28.08.2015 DE 102015216469
(43) Veröffentlichungstag der Anmeldung: 04.07.2018
(73) Patentinhaber: SIMEX Medizintechnik GmbH, 78652 Deißlingen (DE)
(72) Erfinder: SIRING, Rainer, 78652 Deißlingen (DE)
(74) Vertreter: FDST Patentanwälte
(86) Internationale Anmeldenummer: PCT/EP2016/069850
(87) Internationale Veröffentlichungsnummer: WO 2017/036846

(56) Entgegenhaltungen:
- WO-A1-2013/012774
- US-A1- 2014 235 960

## Beschreibung

Die Erfindung betrifft eine Magensonde, die über die Nase eines Patienten einsetzbar ist, mit einem Schlauch, zur nasoenteralen Ernährung des Patienten und/oder zur Ableitung von Magensaft.

Solche Magensonden sind allgemein bekannt. Zur künstlichen Ernährung eines Patienten wird die Magensonde über die Nase und durch den Rachenraum hindurch über die Speiseröhre in den Magen oder sogar bis in den Darm eingesetzt oder auch eingeschoben. Der Schlauch der Magensonde weist zwei Enden auf, ein distales Ende, welches in eingesetztem Zustand im Magen oder im Darm positioniert ist, und ein proximales Ende, welches außerhalb des Patienten positioniert ist und üblicherweise der Nahrungszufuhr dient. Das Verlegen einer Magensonde ist eine übliche Maßnahme in Krankenhäusern und wird auch bei Schlaganfall-Patienten eingesetzt. Aus Untersuchungen ist weiterhin bekannt, dass bei Schlaganfall-Patienten die Gefahr einer Lungenentzündung hoch ist. Eine Magensonde, welche Speichel oder Sekret aus der Speiseröhre absaugen kann, ist bekannt aus WO 2013/012774 A1.

Der Erfindung liegt die Aufgabe zu Grunde, die Gefahr einer Lungenentzündung insbesondere bei Schlaganfall-Patienten zu verringern.

Die Aufgabe wird erfindungsgemäß gelöst durch eine Magensonde mit den Merkmalen gemäß Anspruch 1. Vorteilhafte Ausgestaltungen, Weiterbildungen und Varianten sind Gegenstand der Unteransprüche.

Die Magensonde ist über die Nase eines Patienten einsetzbar und weist einen Schlauch zur nasoenteralen Ernährung des Patienten und/oder zur Ableitung von Magensaft auf. An dem Schlauch ist zusätzlich ein Absaugschlauch befestigt, welcher an einem distalen Ende einen Absaugabschnitt mit einer Anzahl von Öffnungen aufweist. Der Absaugabschnitt ist dabei bezüglich des Schlauches zur Ernährung derart angeordnet dass er in einem eingesetzten Zustand im Rachen, d.h. Rachenraum des Patienten platziert ist, zur Absaugung von Speichel oder Sekret aus dem Rachen.

Die mit der Erfindung erzielten Vorteile bestehen insbesondere darin, dass die Magensonde mit einem zusätzlichen Absauglumen und damit einer Absaugmöglichkeit ausgestattet ist, um ein ungewolltes Eindringen von Speichel in die Luftröhre wirkungsvoll zu verhindern. Der Speichel wird daher vorteilhaft vor Erreichen der Luftröhre über den Absaugabschnitt abgeführt. Der Absaugschlauch ist dazu derart an dem Schlauch befestigt, dass in eingesetztem Zustand der Magensonde der Absaugabschnitt des Absaugschlauchs im Rachen des Patienten platziert ist, d.h. pharyngeal und insbesondere nahe oder noch vor dem Kehlkopf. Eine Absaugung von Speichel erfolgt dann besonders effektiv vor der Verzweigung in Luft- und Speiseröhre. Insgesamt wird dadurch das Risiko einer Lungenentzündung bei einem mit einer Magensonde intubierten Patienten deutlich verringert.

Diese Ausgestaltung beruht dabei auf der Überlegung, dass bei Schlaganfall-Patienten häufig Schluckstörungen auftreten, mit einer Gefahr der Aspiration, d.h. der Gefahr des Eindringens von Speichel und generell Sekreten und/oder Partikeln in die Luftröhre. Diese gelangen dann über die Luftröhre in die Lunge und können dort Ursache der Lungenentzündung sein. Weiterhin beruht dies auf der Erkenntnis, dass Schluckstörungen bei Schlaganfall-Patienten häufig nicht erkannt werden, da deren Diagnose typischerweise einen Spezialisten erfordert, welcher oft nicht zur Verfügung steht.

Mit der hier vorgeschlagenen Lösung wird daher sichergestellt, dass bei künstlich ernährten Schlaganfall-Patienten die Gefahr einer Lungenentzündung verringert ist. Eine explizite Diagnose der Schluckstörungen ist dabei nicht erforderlich. Der besondere Vorteil der hier beschriebenen Magensonde ist u.a. auch darin zu sehen, das quasi eine automatische vorsorgliche Absaugung erfolgen kann, ohne das spezielle Diagnosen oder spezielle weitere Maßnahmen erforderlich sind.

Besonders vorteilhaft ist dabei weiterhin, dass zum Einen keine zusätzliche orale Absaugung notwendig ist und der Mundraum entsprechend freigehalten wird; zum Anderen, dass auch auf eine Intubation oder eine Tracheotomie zur Vermeidung einer Aspiration, d.h. auf einen chirurgischen Eingriff verzichtet werden kann.

Bei Patienten, die beatmet werden, werden häufig sogenannten Trachealkanülen eingesetzt, wie sie beispielsweise aus der WO 2011/054507 A1 bekannt sind. Die Trachealkanüle wird hierbei über einen Luftröhrenschnitt in die Luftröhre eingeführt. Zum Verhindern einer Aspiration bei derartigen Trachealkanülen ist es bekannt, die Trachealkanüle mit einer Absaugung zu versehen und an der Trachealkanüle einen sogenannten Cuff anzubringen, der für eine Abdichtung sorgt, so dass kein Sekret in die Lunge gelangen kann.

Mit der hier beschriebenen Magensonde erfolgt auf besonders einfache und kompakte Weise sowohl die künstliche Ernährung wie auch die Absaugung über die Nase. Mit anderen Worten: mittels der Magensonde ist eine nasoenterale Nahrungszufuhr mit einer nasopharyngalen Absaugung kombiniert.

Der Schlauch der Magensonde dient vorrangig der Zufuhr von Nahrung oder auch Medikamenten in den Verdauungstrakt. Dazu ist der Schlauch entsprechend dimensioniert und weist ein distales Ende auf, welches in eingesetztem Zustand der Magensonde im Magen oder im Darm positioniert ist und eine Öffnung zur Abgabe der über den Schlauch transportierten Stoffe aufweist. Weiterhin weist der Schlauch ein proximales Ende auf, welches auch nach dem Einsetzen außerhalb des Patienten verbleibt und vorrangig zur Zuführung von Nahrung verwendet wird. In einer Variante weist der Schlauch mehrere proximale Enden auf, zur Zuführung verschiedener Stoffe oder zur Realisierung von Zusatzfunktionen, wie beispielsweise einer Ableitung von Magensaft.

Der Absaugschlauch ist mit dem Schlauch verbunden und an diesem befestigt, beispielsweise angeklebt oder ist einstückig mit diesem ausgebildet, beispielsweise durch eine gemeinsame Extrusion. Dabei erstreckt sich der Absaugschlauch im Wesentlichen in der gleichen Richtung wie der Schlauch. Insgesamt ist dadurch eine besonders sichere Positionierung des Absaugschlauchs beim Einsetzen gewährleistet. Der Absaugschlauch und der Schlauch weisen zudem jeweils einen Innenraum oder auch ein Lumen auf, wobei die beiden Innenräume nicht miteinander verbunden sondern voneinander getrennt sind, sodass kein Austausch zwischen dem Absaugschlauch und dem Schlauch erfolgt.

Zum Einsetzen wird die Magensonde beginnend mit dem distalen Ende über die Nase in den Rachenraum eingeschoben. Der Schlauch wird dann weiter am Kehlkopf vorbei und durch die Speiseröhre in den Magen oder bis in den Darm vorgeschoben. Der Absaugschlauch ist dagegen kürzer als der Schlauch und dringt nicht bis in die Speiseröhre ein, sondern endet vielmehr im Rachen oder kurz dahinter, d.h. insbesondere maximal kurz hinter dem Kehlkopf oder noch davor. Auf diese Weise ist der Absaugabschnitt, welcher am distalen Ende des Absaugschlauchs angeordnet ist, auf optimale Weise zur Absaugung im Rachen positioniert. Die Längen des Schlauches und des Absaugschlauches sowie die Befestigungsposition des Absaugschlauches am Schlauch sind daher derart gewählt, dass im eingesetzten Zustand das distale Ende des Schlauches für die Ernährung an der vorgesehenen Position am Magen und das distale Ende des Absaugschlauches oberhalb des Kehlkopfes endet. Ausgehend von einem gemeinsamen Startpunkt vor oder in der Nase ist der Schlauch somit etwa um die Länge der Speiseröhre länger als der Absaugschlauch, zuzüglich etwaiger zusätzlicher Längen im Verdauungstrakt.

Im Querschnitt weist der Schlauch insbesondere eine Querschnittsfläche im Bereich von etwa 1,7 bis 22 mm² auf. Dies entspricht bei einem kreisrunden Querschnitt einem Durchmesser im Bereich von etwa 1,5 bis 5,3 mm. Besonders bevorzugt ist ein Durchmesser im Bereich von 2 bis 4 mm. Der Absaugschlauch weist im Querschnitt insbesondere eine Querschnittsfläche im Bereich von etwa 0,25 bis 20 mm² auf. Dies entspricht bei einem kreisrunden Querschnitt einem Durchmesser im Bereich von etwa 0,6 bis 5 mm.

Die Öffnungen auf dem Absaugabschnitt dienen vorrangig der Aufnahme von Flüssigkeit, insbesondere Speichel oder Sekreten im Rachen, d.h. Rachenraum. In einer ersten Variante ist zumindest endseitig des Absaugschlauchs, d.h. an einer Stirnfläche am distalen Ende, eine Öffnung vorhanden. Mit anderen Worten: der Absaugschlauch ist in Längsrichtung offen.

Bevorzugterweise sind die Öffnungen zusätzlich, vorzugsweise alternativ in radialer Richtung in eine Wandung des Absaugschlauchs eingebracht. Allgemein ist vorzugsweise eine Vielzahl von vergleichsweise kleinen Öffnungen auf dem Absaugabschnitt angeordnet, wobei unter vergleichsweise klein vorzugsweise verstanden wird, dass eine jeweilige Öffnung einen Durchmesser im Bereich von etwa 0,1 bis 4 mm aufweist, besonders bevorzugt im Bereich von 1 bis 3 mm. Die Öffnungen sind dabei nicht notwendigerweise kreisrund, sondern beispielsweise oval. Der Durchmesser der Öffnung ist dann insbesondere in Richtung der größten Ausdehnung der Öffnung gemessen. Insbesondere sind die Öffnungen auf den Absaugabschnitt begrenzt. Oberhalb des Absaugabschnitts sind daher keine weiteren Öffnungen mehr ausgebildet.

Die Öffnungen in der Wandung des Absaugschlauches sind dabei bevorzugt gleichmäßig verteilt angeordnet und weisen auch identische Durchmesser auf. Hierdurch wird eine homogene Absaugung über die gesamte Länge des Absaugabschnitts gewährleistet.

In einer geeigneten Variante ist der Absaugschlauch durch eine abschnittsweise doppelwandige Ausgestaltung der Magensonde ausgebildet und die Öffnungen sind in eine Außenwand eingebracht. Mit anderen Worten: die Magensonde ist zum proximalen Ende hin auf einem bestimmten Abschnitt doppelwandig ausgeführt, wobei eine Innenwand den Innenraum des Absaugschlauchs vom Innenraum des Schlauchs trennt und eine Außenwand den Innenraum des Absaugschlauchs gegenüber der Umgebung begrenzt. Durch diese Integration des Absaugschlauchs ist eine besonders kompakte Magensonde realisiert. In einer Ausgestaltungsform weist der Absaugschlauch dann am distalen Ende keine Stirnfläche auf.

Grundsätzlich sind dabei diverse Ausführungen denkbar. In einer ersten Variante ist die Außenwand quasi auf den Schlauch aufgebaut, sodass der Absaugschlauch in radialer Richtung vom Schlauch absteht und eine Verdickung der Magensonde bildet. In einer zweiten Variante ist der Absaugschlauch in den Innenraum des Schlauchs integriert, indem die Innenwand einen Teilraum abgrenzt. In dieser Ausgestaltung weist die Magensonde entlang des Schlauchs insbesondere einen konstanten Durchmesser auf. Der Absaugschlauch führt dann zu einer abschnittsweisen Verjüngung des Schlauchs. In einer dritten Variante oder zusätzlich zu einer der vorgenannten Varianten ist der Absaugschlauch ringförmig ausgeführt, sodass dessen Innenraum den Schlauch vollumfänglich umschließt. Beispielsweise sind hierzu die Innen- und die Außenwand konzentrisch zueinander angeordnet. In jedem Fall sind die Öffnungen auf dem Ansaugabschnitt lediglich in die Außenwand eingebracht, sodass lediglich Speichel, Sekrete und Partikel von außen in den Ansaugschlauch gelangen.

Alternativ zu der dopppelwandigen Ausgestaltung ist der Absaugschlauch als separater Schlauch am Ernährungsschlauch angeordnet, so dass die beiden Schläuche im Querschnitt betrachtet die Form nach Art einer liegenden Acht aufweisen. Dabei weist der Absaugschlauch dann insbesondere einen geringeren Durchmesser auf als der Schlauch, die beiden Schleifen der Acht sind also nicht gleich groß. Der Absaugschlauch ist beispielsweise am Schlauch durch Kleben oder Schweißen stoffschlüssig mit dem Schlauch verbunden. Alternativ ist einer mit dem Schlauch einstückig, beispielsweise in einem gemeinsamen Extrusionsverfahren ausgebildet, bei dem die beiden Schläuche mit gemeinsamer Wandung ausgebildet werden.

In einer zweckmäßigen Ausgestaltung sind die Öffnungen als Perforation des Absaugschlauchs ausgebildet. Eine solche Perforation gewährleistet vorteilhaft einen reduzierten Druckverlust und somit ein optimales Ansaugen. Bei der Perforation beträgt der Durchmesser einer jeweiligen Öffnung vorzugsweise etwa 0,1 bis 2 mm. Desweiteren sind die Öffnungen mit einer bestimmten Dichte auf dem Ansaugabschnitt verteilt und die Dichte beträgt vorzugsweise etwa 1 bis 4 Öffnungen pro Zentimeter entlang der Länge des Absaugschlauchs. Beispielsweise sind auf einem 3 cm langen Absaugabschnitt 3 bis 10 Öffnungen mit einem Durchmesser von jeweils etwa 1,2 mm angebracht. Der Durchmesser des Absaugschlauchs beträgt hierbei beispielsweise 3 mm.

Die Dimensionierung der Magensonde ist grundsätzlich von der Anatomie des Patienten abhängig. Um eine optimale Positionierung des Absaugbereichs im Rachen des Patienten zu gewährleisten weist der Absaugschlauch zweckmäßigerweise eine Länge von etwa 20 bis 25 cm auf. Der Schlauch ist dagegen deutlich länger und ist insbesondere etwa zwei- bis dreimal so lang. In einer Variante ist der Schlauch bis zu fünfmal so lang, insbesondere bei Verwendung desselben als Duodenalsonde. Auf diese Weise ist sichergestellt, dass in eingesetztem Zustand das distale Ende des Schlauchs im Verdauungstrakt positioniert ist, während der Absaugabschnitt im Rachen positioniert ist.

Der Absaugabschnitt weist bevorzugterweise eine Länge im Bereich von 1 bis 10 cm auf und ist dadurch dann optimal zur Absaugung von Speichel entlang des Rachens dimensioniert. Besonders geeignet ist dabei eine Länge von etwa 2,5 cm.

In einer besonders bevorzugten Ausgestaltung ist der Absaugabschnitt ablängbar und weist dadurch insbesondere eine veränderbare Länge auf. Da die distalen Enden des Schlauchs und des Absaugschlauchs im Wesentlichen durch die Länge der Speiseröhre voneinander beabstandet sind, diese jedoch je nach Patient unterschiedlich lang sein kann, ist es mittels des ablängbaren Absaugabschnitts dann möglich, auch einen Abstand zwischen den beiden distalen Enden optimal einzustellen. Dazu wird dann der Absaugabschnitt und somit auch der Absaugschlauch entsprechend abgelängt, d.h. verkürzt, und dazu beispielsweise abgeschnitten.

Im Zusammenhang mit dem Ablängen ist insbesondere eine bestimmte Mindestlänge des Absaugabschnitts sinnvoll, um zu gewährleisten, dass auch bei besonders kleinen Patienten und einer entsprechend starken Kürzung des Ansaugabschnitts noch genügend Öffnungen vorhanden sind und eine hinreichende Absaugung sichergestellt ist. Eine geeignete Mindestlänge ist hierbei insbesondere 8 cm.

Vorteilhafterweise ist der Absaugabschnitt vom Schlauch lösbar, wodurch insbesondere ein Ablängen des Absaugabschnitts deutlich vereinfacht wird und eine versehentliche Beschädigung des Schlauchs vorteilhaft verhindert wird. Der Absaugschlauch ist vorzugsweise nicht entlang dessen gesamter Länge am Schlauch befestigt, sondern lediglich abschnittsweise, sodass der Absaugabschnitt zumindest teilweise oder auch vollständig vom Schlauch quasi abgeklappt werden kann. Um dennoch eine optimale Positionierung des Absaugabschnitts zu gewährleisten und insbesondere ein versehentliches Wegklappen vom Schlauch beim Einsetzen zu vermeiden, sind der Schlauch und der Absaugschlauch geeigneterweise derart ausgeführt, dass diese aneinander haften, d.h. lösbar miteinander verbunden sind, beispielsweise über Adhäsionskräfte aufgrund geeignet ausgewählter Materialien Oberflächenbehandlungen oder auch aufgrund geeigneter (Klebe-)Zwischenschichten. Alternativ ist diese lösbare Verbindung über geeignete Adhäsionskräfte über die gesamte Befestigungslänge des Absaugschlauchs ausgebildet. Dabei wird vorteilhafterweise ausgenutzt, dass beim Einsetzen durch die Nase der Schlauch und der Absaugschlauch aufgrund der generell geringen Dimensionen innerhalb der Nase gegeneinandergedrückt werden und dann im weiteren Verlauf zusammenbleiben.

Allgemein bestehen besonders die mit dem Patienten in Berührung kommenden Teile der Magensonde zweckmäßigerweise jeweils aus biokompatiblen Materialien, insbesondere biokompatiblen Kunststoffen.

In einer besonders bevorzugten Ausgestaltung ist auf dem Absaugschlauch an einem Ende eine Anzahl von Längenmarkierungen angebracht, welche jeweils einen Abstand zum anderen Ende des Absaugschlauchs angeben. In einer ersten Variante geben dabei die Längenmarkierungen einen Abstand zum distalen Ende an, sodass beim Einsetzen anhand der Längenmarkierungen abgelesen werden kann, wie weit das distale Ende des Absaugschlauchs im Speziellen und die Magensonde im Allgemeinen bereits eingeschoben ist.

In einer bevorzugten zweiten Variante sind die Längenmarkierungen auf dem Absaugabschnitt angebracht und der Absaugabschnitt ist an den Längenmarkierungen ablängbar, wodurch dann der Absaugabschnitt auf besonders einfache Weise konfektionierbar ist. In dieser Variante geben die Längenmarkierungen insbesondere jeweils einen Abstand zum proximalen Ende des Absaugschlauchs an. Dieser wird dann in Abhängigkeit der konkreten Anatomie des jeweiligen Patienten auf die passende Länge abgelängt.

Bei der Magensonde wird entlang des Absaugschlauchs und im Bereich des proximalen Endes des Absaugschlauchs zweckmäßigerweise auf einen sogenannten Cuff verzichtet, wie er bei Trachealkanülen bekannt ist. Dabei wird unter Cuff insbesondere ein aufblasbarer Balg verstanden, der regelmäßig zur formschlüssigen Abdichtung bei in Hohlräumen verlegten Schläuchen verwendet wird. Aufgrund der Absaugung von Speichel im Bereich des Rachens und insbesondere auch des Kehlkopfs ist es insbesondere möglich auf ein Verschließen der Luftröhre mittels eines Cuffs zu verzichten, weshalb bevorzugterweise entsprechend auf die Anordnung eines Cuffs auf proximaler Seite, d.h. oberhalb des Kehlkopfs oder vor diesem verzichtet wird.

Weiterhin denkbar ist dagegen die Verwendung eines Cuffs in der Speiseröhre, um beispielsweise ein Aufsteigen von Flüssigkeiten aus dem Magen zu verhindern. Bevorzugterweise in einer zweckmäßigen Ausgestaltung der Magensonde vollständig auf einen Cuff verzichtet, die Magensonde also insgesamt frei von einem Cuff ist.

Insbesondere am proximalen Ende weist der Absaugschlauch geeigneterweise einen Anschluss auf, zum Anschließen einer Saugpumpe, wodurch auf einfache Weise ein insbesondere automatisches Absaugen ermöglicht ist. Der Anschluss ist dann beispielsweise ein Stecker oder Teil einer Kupplung zur Verbindung mit einem Anschlussschlauch. Ein entsprechendes Ernährungs-System umfasst daher neben der Magensonde auch eine Absaugvorrichtung, die am Absaugschlauch anschließbar und insbesondere auch angeschlossen ist.

Um insbesondere die Handhabung der Magensonde zu vereinfachen zweigt der Absaugschlauch zweckmäßigerweise am proximalen Ende vom Schlauch ab. Dadurch sind dann insbesondere die Zugänge über die proximalen Enden des Schlauchs und des Absaugschlauchs räumlich voneinander unabhängig positionierbar und dadurch wiederum einfacher an jeweilige Anschlüsse anschließbar. Die Magensonde weist dann entsprechend eine Abzweigung auf, an welcher das proximale Ende vom Schlauch abzweigt und dann insbesondere auch nicht mit dem Schlauch verbunden ist.

Nachfolgend werden Ausführungsbeispiele anhand einer Zeichnung näher erläutert. Darin zeigen jeweils schematisch:
- Fig. 1: eine erste Variante einer Magensonde, und
- Fig. 2: eine zweite Variante der Magensonde.

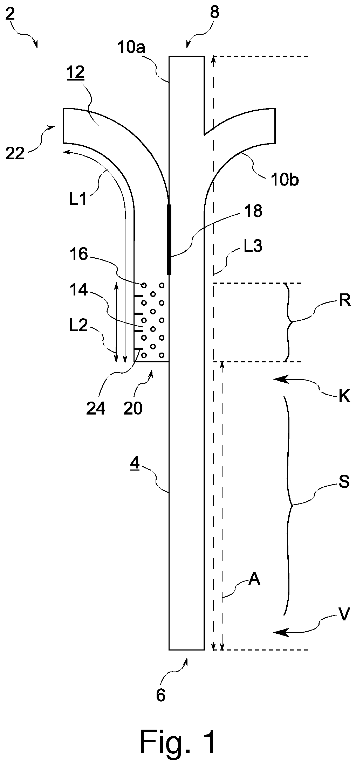

Fig. 1 zeigt eine erste Variante einer Magensonde 2, welche zur nasoenteralen Ernährung eines Patienten über dessen Nase einsetzbar ist. Hierzu weist die Magensonde 2 einen Schlauch 4 mit einem distalen Ende 6 auf, welches in eingesetztem Zustand im Magen oder im Darm des Patienten positioniert ist. Weiterhin weist der Schlauch 4 ein proximales Ende 8 auf, welches in eingesetztem Zustand außerhalb des Patienten verbleibt. Zur künstlichen Ernährung wird dann vom proximalen Ende 8 aus beispielsweise Nahrung zugeführt. Dabei weist der Schlauch 4 in der hier gezeigten Ausführungsform am proximalen Ende 8 zwei Zugänge 10a, 10b auf, welche zur Zuführung unterschiedlicher Stoffe verwendet werden können. Beispielsweise ist der eine Zugang 10a zum Anschließen einer Spritze mit Nahrung ausgebildet und der andere Zugang 10b als Spülkanal.

Zusätzlich zum Schlauch 4 zur nasoenteralen Ernährung weist die Magensonde 2 einen Absaugschlauch 12 auf, zur nasopharyngalen Absaugung von Speichel aus dem Rachen des Patienten. Der Absaugschlauch 12 weist hierzu einen Absaugabschnitt 14 auf, auf welchem eine Anzahl von Öffnungen 16 in den Absaugschlauch 12 eingebracht ist. Der Absaugschlauch 12 und der Absaugabschnitt 14 sind zudem auf einem Abschnitt 18 am Schlauch 4 befestigt und dadurch relativ zu diesem fix positioniert. Am proximalen Ende 8 des Schlauchs 4 zweigt der Absaugschlauch 12 vom Schlauch 4 ab.

Um in eingesetztem Zustand der Magensonde 2 den Absaugabschnitt 14 im Rachen zu positionieren, ist der Absaugschlauch 12 deutlich kürzer ausgeführt als der Schlauch 4, sodass der Absaugschlauch 12 in eingesetztem Zustand deutlich höher endet, als der Schlauch 4. In eingesetztem Zustand liegt dann das distale Ende 6 der Magensonde 2 im Vertauungstrakt des Patienten, während der Absaugabschnitt 14 im Rachen des Patienten positioniert ist. Der Absaugschlauch 12 weist dann ein distales Ende 20 auf, welches zum distalen Ende 6 des Schlauchs 4 in einem Abstand A beabstandet ist.

Zur Verdeutlichung der Positionierung der Magensonde 2 in eingesetztem Zustand ist in Fig. 1 die jeweilige Lage des Rachens R, des Kehlkopfs K, der Speiseröhre S und des Verdauungstrakts V des Patienten angedeutet. Deutlich erkennbar ist, dass der Schlauch 4 bis in den Verdauungstrakt V vordringt, der Absaugschlauch 12 jedoch lediglich bis maximal an den Kehlkopf K heranreicht, sodass der Absaugabschnitt 14 durch den Rachen R geführt ist.

Dadurch ist auf effektive Weise eine Aspiration, das heißt das Eindringen von Speichel und allgemeinen Flüssigkeiten und/oder Partikeln in die Luftröhre verhindert, welche nicht dargestellt ist, allerdings unterhalb des Kehlkopfs K beginnt. Denkbar ist generell auch eine Ausführung, bei welcher der Absaugabschnitt 14 leicht über den Kehlkopf hinausgeht, jedoch insbesondere nicht mehr als etwa 1 cm, um im kompletten Rachen R abzusaugen.

Der Absaugschlauch 12 weist eine Länge L1 von etwa 20 bis 25 cm auf, wobei der Absaugabschnitt 14 am distalen Ende 20 des Absaugschlauchs 12 eine Länge L2 von etwa 5 bis 10 cm, insbesondere 8 cm aufweist. Der Schlauch 4 weist dagegen eine Länge L3 auf, welche etwa das Zwei- bis Dreifache der Länge L1 des Absaugschlauchs 12 entspricht.

In der in Fig. 1 gezeigten Ausführungsform sind auf dem Absaugabschnitt 14 zudem mehrere Längenmarkierungen 24 angebracht, beispielsweise als farbige Markierungen aufgedruckt. Diese Markierungen geben hier den Abstand zu einem proximalen Ende 22 des Absaugschlauchs 12 an. Um nun die beiden distalen Enden 6, 20 optimal voneinander zu beabstanden, ist der Absaugabschnitt 14 hier ablängbar ausgebildet, und zwar insbesondere an den Längenmarkierungen 24. Um dann den besonderen individuellen anatomischen Gegebenheiten bei einem bestimmten Patienten Rechnung zu tragen, wird vor dem Einsetzen der Magensonde 2 der Absaugschlauch 12 entsprechend geeignet abgelängt und ein optimaler Abstand A zwischen den beiden distalen Enden 6, 20 eingestellt.

Um insbesondere das Ablängen zu vereinfachen ist der Absaugschlauch 12 in Fig. 1 teilweise vom Schlauch 4 lösbar, und zwar entlang des Absaugabschnitts 14. Dieser kann dann einfach vom Schlauch 4 abgeklappt, abgesetzt oder abgehoben und dann abgelängt werden, ohne dabei den Schlauch 4 zu beschädigen.

Der Absaugschlauch 12 weist weiterhin an dessen proximalem Ende 22 einen nicht näher dargestellten Anschluss auf, an welchen zum Absaugen eine Saugpumpe angeschlossen werden kann,

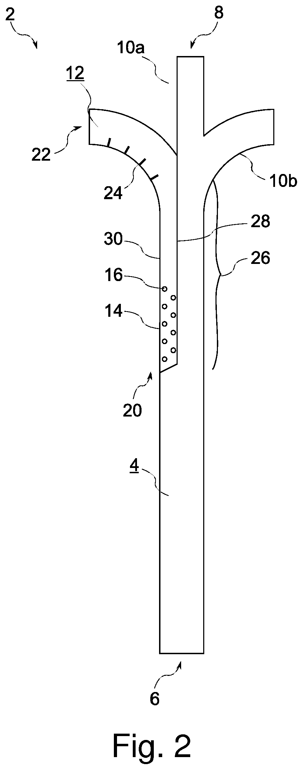

Fig. 2 zeigt eine zweite Variante der Magensonde 2, bei welcher der Absaugschlauch 12 durch eine doppelwandige Ausgestaltung des Schlauchs 4 ausgebildet ist. In dieser doppelwandigen Ausgestaltung weist der Schlauch 4 einen doppelwandigen Abschnitt 26 auf, auf welchem eine Innenwand 28 den Schlauch 4 vom Absaugschlauch 12 trennt. Eine Außenwand 30 des doppelwandigen Abschnitts 26 bildet dann eine Abgrenzung des Absaugschlauchs 12 gegen die Umgebung. Auf dieser Außenwand 30 sind dann auch die Öffnungen 16 angeordnet.

Bei dem in Fig. 2 gezeigten Ausführungsbeispiel sind zudem am proximalen Ende 22 des Absaugschlauchs 12 Längenmarkierungen 24 angebracht, welche den Abstand der jeweiligen Längenmarkierung 24 zum distalen Ende 20 des Absaugschlauchs 12 hin angeben. Auf diese Weise ist es dann möglich, beim Einschieben der Magensonde 2 in den Patienten anhand der Längenmarkierungen 24 abzulesen, wie weit die Magensonde 2 bereits vorgeschoben ist.

Die beiden gezeigten Varianten der Magensonde 2 sind jeweils frei von einem Cuff. Ein solcher wird aufgrund der speziellen Ausgestaltung zunächst auch nicht benötigt, da durch die Positionierung des Absaugabschnitts 14 im Rachen R die Gefahr einer Aspiration bereits deutlich reduziert ist. Ein Verschließen des Rachens R oder der Speiseröhre S mittels eines Cuffs erfolgt daher nicht. In einer nicht gezeigten Alternative ist jedoch im Bereich der Speiseröhre S am Schlauch 4 ein Cuff angebracht.

### Bezugszeichenliste

- 2: Magensonde
- 4: Schlauch
- 6: distales Ende des Schlauchs
- 8: proximales Ende des Schlauchs
- 10a,10b: Zugang
- 12: Absaugschlauch
- 14: Absaugabschnitt
- 16: Öffnung
- 18: Abschnitt
- 20: distales Ende des Absaugschlauchs
- 22: proximales Ende des Absaugschlauchs
- 24: Längenmarkierung
- 26: doppelwandiger Abschnitt
- 28: Innenwand
- 30: Außenwand

- A: Abstand
- R: Rachen
- K: Kehlkopf
- S: Speiseröhre
- V: Verdauungstrakt
- L1, L2, L3: Länge

## Patentansprüche

1. Magensonde (2), die über die Nase eines Patienten einsetzbar ist, mit einem Schlauch (4), zur nasoenteralen Ernährung des Patienten und/oder zur Ableitung von Magensaft,
**dadurch gekennzeichnet,**
**dass** an dem Schlauch (4) ein Absaugschlauch (12) befestigt ist, welcher an einem distalen Ende (20) einen Absaugabschnitt (14) mit einer Anzahl von Öffnungen (16) aufweist, und welcher derart angeordnet ist, dass der Absaugabschnitt (14) in einem eingesetzten Zustand im Rachen (R) des Patienten platziert ist, zur Absaugung von Speichel aus dem Rachen (R).

2. Magensonde (2) nach dem vorhergehenden Anspruch,
**dadurch gekennzeichnet,**
**dass** der Absaugschlauch (12) durch eine abschnittsweise doppelwandige Ausgestaltung der Magensonde (2) ausgebildet ist und die Öffnungen (16) in eine Außenwand (30) eingebracht sind.

3. Magensonde (2) nach einem der vorhergehenden Ansprüche,
**dadurch gekennzeichnet,**
**dass** die Öffnungen (16) als Perforation des Absaugschlauchs (12) ausgebildet sind.

4. Magensonde (2) nach einem der vorhergehenden Ansprüche,
**dadurch gekennzeichnet,**
**dass** der Absaugschlauch (12) eine Länge (L1) von etwa 20 bis 25 cm aufweist.

5. Magensonde (2) nach einem der vorhergehenden Ansprüche,
**dadurch gekennzeichnet,**
**dass** der Absaugabschnitt (14) eine Länge (L2) im Bereich von 1 bis 10 cm aufweist, bevorzugt etwa 2,5 cm.

6. Magensonde (2) nach einem der vorhergehenden Ansprüche,
**dadurch gekennzeichnet,**
**dass** der Absaugabschnitt (14) ablängbar ist.

7. Magensonde (2) nach einem der vorhergehenden Ansprüche,
**dadurch gekennzeichnet,**
**dass** der Absaugabschnitt (14) vom Schlauch (4) lösbar ist.

8. Magensonde (2) nach einem der vorhergehenden Ansprüche,
**dadurch gekennzeichnet,**
**dass** auf dem Absaugschlauch (12) an einem Ende (6, 8) eine Anzahl von Längenmarkierungen (24) angebracht ist, welche jeweils einen Abstand zum anderen Ende (8, 6) des Absaugschlauchs (12) angeben.

9. Magensonde (2) nach dem vorhergehenden Anspruch,
**dadurch gekennzeichnet,**
**dass** die Längenmarkierungen (24) auf dem Absaugabschnitt (14) angebracht sind und der Absaugabschnitt (14) an den Längenmarkierungen (24) ablängbar ist.

10. Magensonde (2) nach einem der vorhergehenden Ansprüche,
**dadurch gekennzeichnet,**
**dass** diese entlang des Absaugschlauchs (12) und im Bereich eines proximalen Endes (22) des Absaugschlauchs (12) frei von einem Cuff ist.

11. Magensonde (2) nach einem der vorhergehenden Ansprüche,
**dadurch gekennzeichnet,**
**dass** der Absaugschlauch (12) einen Anschluss aufweist, zum Anschließen einer Saugpumpe.

12. Magensonde (2) nach einem der vorhergehenden Ansprüche,
**dadurch gekennzeichnet,**
**dass** der Absaugschlauch (12) am proximalen Ende (8) vom Schlauch (4) abzweigt.

## Claims

1. Gastric probe (2) which can be inserted via the nose of a patient, with a feed hose (4) for nasoenteral feeding of the patient and/or for removal of gastric juice, **characterized in that** a suction hose (12) which is secured to the feed hose (4) has, at a distal end (20), a suction portion (14) with a number of openings (16) and is arranged in such a way that, in an inserted state, the suction portion (14) is placed in the pharynx (R) of the patient in order to suction saliva from the pharynx (R).

2. Gastric probe (2) according to the preceding claim, **characterized in that** the suction hose (12) is formed by the gastric probe (2) being configured in part with a double wall, and the openings (16) are introduced into an outer wall (30) .

3. Gastric probe (2) according to one of the preceding claims, **characterized in that** the openings (16) are configured as a perforation of the suction hose (12).

4. Gastric probe (2) according to one of the preceding claims, **characterized in that** the suction hose (12) has a length (L1) of approximately 20 to 25 cm.

5. Gastric probe (2) according to one of the preceding claims, **characterized in that** the suction portion (14) has a length (L2) in the range of 1 to 10 cm, preferably approximately 2.5 cm.

6. Gastric probe (2) according to one of the preceding claims, **characterized in that** the suction portion (14) can be cut to length.

7. Gastric probe (2) according to one of the preceding claims, **characterized in that** the suction portion (14) is releasable from the feed hose (4).

8. Gastric probe (2) according to one of the preceding claims, **characterized in that** a number of length markings (24) are applied at an end (6, 8) of the suction hose (12) and in each case indicate a distance to the other end (8, 6) of the suction hose (12).

9. Gastric probe (2) according to the preceding claim, **characterized in that** the length markings (24) are applied to the suction portion (14), and the suction portion (14) can be cut to length at the length markings (24).

10. Gastric probe (2) according to one of the preceding claims, **characterized in that** it is free of a cuff along the suction hose (12) and in the region of a proximal end (22) of the suction hose (12) .

11. Gastric probe (2) according to one of the preceding claims, **characterized in that** the suction hose (12) has an attachment piece for the attachment of a suction pump.

12. Gastric probe (2) according to one of the preceding claims, **characterized in that** the suction hose (12) branches off from the feed hose (4) at the proximal end (8).

## Revendications

1. Sonde gastrique (2), qui peut être introduite via le nez d'un patient, avec un tuyau souple (4), pour la nutrition nasogastrique du patient et/ou pour l'évacuation de suc gastrique, **caractérisée en ce qu'**un tuyau d'aspiration (12) est fixé au tuyau souple (4), présente à une extrémité distale (20) une section d'aspiration (14) avec un nombre d'ouvertures (16), et est disposé de telle manière que la section d'aspiration (14) dans l'état introduit soit placée dans la gorge (R) du patient pour aspirer de la salive hors de la gorge (R).

2. Sonde gastrique (2) selon la revendication précédente, **caractérisée en ce que** le tuyau d'aspiration (12) est formé par une configuration localement à double paroi de la sonde gastrique (2) et les ouvertures (16) sont pratiquées dans une paroi extérieure (30).

3. Sonde gastrique (2) selon l'une quelconque des revendications précédentes, **caractérisée en ce que** les ouvertures (16) sont formées par des perforations du tuyau d'aspiration (12).

4. Sonde gastrique (2) selon l'une quelconque des revendications précédentes, **caractérisée en ce que** le tuyau d'aspiration (12) présente une longueur (L1) d'environ 20 à 25 cm.

5. Sonde gastrique (2) selon l'une quelconque des revendications précédentes, **caractérisée en ce que** la section d'aspiration (14) présente une longueur (L2) située dans la plage de 1 à 10 cm, de préférence d'environ 2,5 cm.

6. Sonde gastrique (2) selon l'une quelconque des revendications précédentes, **caractérisée en ce que** la section d'aspiration (14) peut être coupée à longueur.

7. Sonde gastrique (2) selon l'une quelconque des revendications précédentes, **caractérisée en ce que** la section d'aspiration (14) peut être détachée du tuyau souple (4).

8. Sonde gastrique (2) selon l'une quelconque des revendications précédentes, **caractérisée en ce qu'**un nombre de marques de longueur (24) sont apposées sur le tuyau d'aspiration (12) à une extrémité (6, 8), et indiquent respectivement une distance jusqu'à l'autre extrémité (8, 6) du tuyau d'aspiration (12).

9. Sonde gastrique (2) selon l'une quelconque des revendications précédentes, **caractérisée en ce que** les marques de longueur (24) sont apposées sur la section d'aspiration (14) et la section d'aspiration (14) peut être coupée à longueur aux marques de longueur (24).

10. Sonde gastrique (2) selon l'une quelconque des revendications précédentes, **caractérisée en ce qu'**elle est dépourvue d'une manchette le long du tuyau d'aspiration (12) et dans la région d'une extrémité proximale (22) du tuyau d'aspiration (12).

11. Sonde gastrique (2) selon l'une quelconque des revendications précédentes, **caractérisée en ce que** le tuyau d'aspiration (12) présente un raccord, pour le raccordement d'une pompe aspirante.

12. Sonde gastrique (2) selon l'une quelconque des revendications précédentes, **caractérisée en ce que** le tuyau d'aspiration (12) dévie du tuyau souple (4) à l'extrémité proximale (8).
